(19) Europäisches Patentamt
European Patent Office
Office européen des brevets

(11) Veröffentlichungsnummer: **0 013 034 B1**

# (12) EUROPÄISCHE PATENTSCHRIFT

(45) Veröffentlichungstag der Patentschrift: **10.11.82**

(51) Int. Cl.³: **C 07 D 307/71**

(21) Anmeldenummer: **79200502.7**

(22) Anmeldetag: **11.09.79**

## (54) Verfahren zur Herstellung von beta-(5-Nitro-2-furyl)-acrolein.

(30) Priorität: **21.12.78 DE 2855245**

(43) Veröffentlichungstag der Anmeldung: **09.07.80 Patentblatt 80/14**

(45) Bekanntmachung des Hinweises auf die Patenterteilung: **10.11.82 Patentblatt 82/45**

(84) Benannte Vertragsstaaten: **AT BE CH DE FR GB IT LU NL**

(56) Entgegenhaltungen:
**GB-A-744 187**

**Chemical abstracts, Band 73, Nr. 11, 14. September 1970, Zusammenfassung Nr. 55962c, Seite 315 Columbus, Ohio, US**

**Die Akte enthält technische Angaben, die nach dem Eingang der Anmeldung eingereicht wurden und die nicht in dieser Patentschrift enthalten sind.**

(73) Patentinhaber: **Rütgerswerke Aktiengesellschaft, Mainzer Landstrasse 217, D-6000 Frankfurt a.Main 1 (DE)**

(72) Erfinder: **Pastorek, Emmerich, Dr., Grünbergerstrasse 90, D-6944 Hemsbach (DE)**
Erfinder: **Orth, Winfried, Dr., Am Schachteigraben 28, D-6733 Hassloch/Pfalz (DE)**
Erfinder: **Fickert, Werner, Dr., Stockacher Strasse 14, D-6800 Mannheim 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von β-(5-Nitro-2-furyl)-acrolein

Die Erfindung betrifft ein Verfahren zur Herstellung von β-(5-Nitro-2-furyl)-acrolein.

β-(5-Nitro-2-furyl)-acrolein dient als Ausgangsstoff zur Herstellung von als Fungizid und Bakterizid wirksamen Substanzen, die in der Pharmazie, Textilindustrie und im Pflanzenschutz Anwendung finden.

Es ist bekannt, β-(5-Nitro-2-furyl)-acrolein durch Kondensation von 5-Nitrofurfural mit Acetaldehyd in Gegenwart eines sekundären Amins herzustellen. Die Reaktion wird in einem inerten organischen Lösungsmittel, wie Benzol, oder auch ohne Lösungsmittel, durchgeführt; die Ausbeuten, die unter Verwendung von Benzol als Lösungsmittel noch am günstigsten liegen, betragen 30–36% (vgl. z.B. DE-AS 1 493 890, US-PS 3 491 123). Die Verwendung von Benzol als Lösungsmittel weist aber, abgesehen von seinen toxischen Eigenschaften, auch noch den Nachteil auf, die bei der Reaktion in grösseren Mengen entstandenen Harze nicht aufzulösen. Dies führt bei der Aufarbeitung zu Schwierigkeiten, wie z.B. Verschmierung der Apparaturen mit den halbfesten Harzen.

Daneben sind noch zahlreiche weitere Verfahren bekannt, die aber auch, insbesondere in bezug auf Ausbeute, Ausgangsmaterial und grosstechnische Durchführbarkeit, Nachteile aufweisen (vgl. z.B. US-PS 2 799 686; JA-PS 15 655(62), ref. in C.A. 59, 9986c; FR-Brevet special de Medicament 1970 M).

Aufgabe der Erfindung war daher ein Verfahren zur Herstellung von β-(5-Nitro-2-furyl)-acrolein, das die geschilderten Nachteile vermeidet und auf einfache, auch grosstechnisch geeignete Weise und mit guter Ausbeute durchführbar ist.

Erfindungsgemäss wird diese Aufgabe durch ein Verfahren zur Herstellung von β-(5-Nitro-2-furyl)-acrolein durch Umsetzung von 5-Nitrofurfural mit Acetaldehyd in Gegenwart von sekundären Aminen gelöst, das dadurch gekennzeichnet ist, dass man die Umsetzung in einer aliphatischen Carbonsäure mit 2 bis 4 Kohlenstoffatomen durchführt. Zweckmässig arbeitet man mit einem Molverhältnis von 5-Nitro-furfural zu Acetaldehyd 1 : 1 bis 1 : 2, insbesondere 1 : 1,6 bis 1 : 1,8 und bei Temperaturen von −10 bis +90 °C, insbesondere bei 10–80 °C.

Als sekundäre Amine werden vorteilhaft Verbindungen der allgemeinen Formel

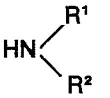 und/oder 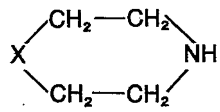

eingesetzt, worin R¹ und R² gleich oder verschieden sind und eine Alkyl-, Hydroxyalkyl-, Alkoxyalkyl-, Alkenyl-, Cycloalkyl- oder Aralkylgruppe bedeuten können und X Sauerstoff, Schwefel, Imino, N-Alkyl oder die Methylengruppe. Ein Heteroatom X kann anstelle in der 4-Stellung auch in der 2- oder 3-Stellung zur NH-Gruppe stehen; es kommen auch an einem Ringkohlenstoffatom substituierte oder analoge 5- oder 7-gliedrige Verbindungen in Betracht.

In diesen Verbindungen besitzt eine Alkyl-, Alkenyl- oder Alkoxyalkylgruppe vorzugsweise 1 bis 7 C-Atome. Sie kann geradkettig oder verzweigt sein und ist z.B. Methyl, Äthyl, Butyl, Isobutyl, Hexyl, Allyl, Hydroxyäthyl, 1-Methyl-2-hydroxy-äthyl, Methoxyäthyl, Äthoxyäthyl, Butyloxyäthyl. Eine Cycloalkylgruppe besitzt vorzugsweise 5–7 C-Atome und ist z.B. Cyclopentyl, Cyclohexyl oder Methylcyclohexyl. In einer Aralkylgruppe ist der aromatische Rest vorzugsweise Phenyl oder auch eine durch Alkyl substituierte Phenylgruppe, eine Ararkylgruppe ist also z.B. Benzyl- oder Phenäthyl.

Die Menge an zugesetztem Amin beträgt zweckmässigerweise 0,02 bis 0,2 Mol pro 1 Mol 5-Nitrofurfural. Aliphatische Carbonsäuren mit 2–4 C-Atomen sind Essigsäure, Propionsäure, Buttersäure oder Isobuttersäure. Man kann die Umsetzung in einer wässrigen Lösung der Carbonsäuren, zweckmässigerweise in einer 20 bis 80%igen wässrigen Lösung, aber auch in den wasserfreien Carbonsäuren durchführen.

Nach dem erfindungsgemässen Verfahren wird β-(5-Nitro-2-furyl)-acrolein in hohen Ausbeuten (45–80% d.Th., bezogen auf eingesetztes 5-Nitrofurfural) erhalten; gegenüber den bekannten, auch von 5-Nitro-furfural und Acetaldehyd ausgehenden Verfahren wird also eine wesentliche Ausbeutesteigerung erzielt. Das erhaltene Produkt kann ohne weitere Reinigung weiterverarbeitet werden, z.B. zu Pharmazeutika.

Unter Prozentangaben sind Gewichtsprozente zu verstehen. Temperaturangaben beziehen sich auf die Celsiusskala.

## Beispiel 1

148,4 g 5-Nitrofurfural, 95%ig, (1 Mol) wird in 400 ml Essigsäure 60%ig, in Stickstoffatmosphäre gelöst, auf 0 °C abgekühlt und 72,8 g Acetaldehyd (1,8 Mol) zugegeben. Nach kurzem Rühren wird bei 5–10 °C innerhalb von 10 Min. unter intensivem Rühren eine Lösung, hergestellt aus 33%iger wässriger Dimethylamin-Lösung (0,088 Mol) in 20 ml Essigsäure, 60%ig, zugetropft. Das Reaktionsgemisch wird kontinuierlich innerhalb von 90 Min. auf 60 °C erhitzt und 150 Min. bei 60–65 °C gerührt. Danach wird das Reaktionsgemisch auf ca. 18 °C abgekühlt, das ausgefallene Produkt abgenutscht und mit wenig Essigsäure, 60%ig, oder Isopropanol nachgewaschen. Nach dem Trocknen werden 88,6 g β-(5-Nitro-2-furyl)-acrolein als gelb bis ocker gefärbtes feinkristallines Produkt erhalten. Die Ausbeute beträgt 88,5 g = 53,0% der Theorie, Fp = 111,5–113,5 °C.

In den Beispielen 2–12 wird wie im Beispiel 1 verfahren, nur werden verschiedene Katalysatoren angewandt. Die Ergebnisse enthält Tabelle 2.

Tabelle 1

| Beispiel | g/Mol Katalysator | Katalysator | Ausbeute in g | in % d. Th. | Fp/°C |
|---|---|---|---|---|---|
| 2 | 12 /0,093 | Dibutylamin | 110 | 65,8 | 113–115 |
| 3 | 12 /0,065 | Dicyclohexylamin | 78 | 46,7 | 114–116 |
| 4 | 7 /0,035 | Dibenzylamin | 83 | 49,6 | 105–110 |
| 5 | 6 /0,057 | Diäthanolamin | 130 | 77,8 | 113–115,5 |
| 6 | 12 /0,090 | Di(2-hydroxypropyl)-amin | 126 | 75,4 | 112–115,5 |
| 7 | 5 /0,066 | Methyl-äthanolamin | 126 | 75,4 | 111–114,5 |
| 8 | 9 /0,067 | Di(methoxyäthanol)-amin | 87 | 52,1 | 107,5–113 |
| 9 | 4 /0,046 | Morpholin | 99 | 59,2 | 110–113 |
| 10 | 6 /0,058 | Thiomorpholin | 105 | 62,8 | 107–111 |
| 11 | 10 /0,112 | Piperidin | 80 | 47,9 | 111–114 |
| 12 | 2,5/0,028 | 1-Methylpiperazin | 91 | 54,5 | 113–115 |

In den Beispielen 13–17 sind Verfahren zur Herstellung von β-(5-Nitro-2-furyl)-acrolein beschrieben, bei denen auf 1 Mol 5-Nitrofurfural 6 g Diäthanolamin/0,057 Mol als Katalysator und 75 g Acetaldehyd/1,7 Mol angewendet werden. Weitere Angaben sind der Tabelle 2 zu entnehmen.

Tabelle 2

| Beispiel | Lösungsmittel | ml | %ig | Reaktion Dauer (Min.) | Temp. °C | Ausbeute g | % d. Th. | Fp/°C |
|---|---|---|---|---|---|---|---|---|
| 13 | Essigsäure | 580 | 30 | 90 | 65 | 78 | 46,7 | 114–116 |
| 14 | Essigsäure | 350 | 99 | 90 | 65 | 130 | 77,8 | 102–110 |
| 15 | Essigsäure | 400 | 60 | 45 | 75–80 | 112 | 67,0 | 114–116 |
| 16 | Propionsäure | 400 | 60 | 120 | 65 | 129 | 77,2 | 108–110 |
| 17 | Isobuttersäure | 450 | 70 | 150 | 65 | 127 | 76,0 | 103–108 |

## Patentansprüche

1. Verfahren zur Herstellung von β-(5-Nitro-2-furyl)-acrolein durch Umsetzung von 5-Nitrofurfural mit Acetaldehyd in Gegenwart eines sekundären Amins, dadurch gekennzeichnet, dass man die Umsetzung in einer aliphatischen Carbonsäure mit 2–4 Kohlenstoffatomen durchführt.

2. Verfahren nach Anspruch 1, dadurch gekennzeichnet, dass das Molverhältnis von 5-Nitrofurfural zu Acetaldehyd 1 : 1 bis 1 : 2 beträgt.

3. Verfahren nach Anspruch 1 oder 2, dadurch gekennzeichnet, dass man bei Temperaturen von −10 bis +90 °C, insbesondere von 10 bis 80 °C arbeitet.

## Claims

1. A process for the production of β-(5-nitro-2-furyl)-acrolein by reacting 5-nitro-furfural and acetaldehyde in the presence of a secondary amine, wherein the reaction is effected in an aliphatic carboxylic acid of 2 to 4 carbon atoms.

2. The process of claim 1 wherein the molar ratio of 5-nitro-furfural to acetaldehyde is 1 : 1 to 1 : 2.

3. The process of claim 1 or 2 wherein the reaction is effected in the temperature range of −10 to +90 °C, especially of 10 to 80 °C.

## Revendications

1. Procédé de préparation de la bêta-(5-nitro-2-furyl)-acroléine par réaction du 5-nitrofurfural avec l'acétaldéhyde en présence d'une amine secondaire, caractérisé en ce que l'on effectue la réaction dans un acide carboxylique aliphatique contenant de 2 à 4 atomes de carbone.

2. Procédé selon la revendication 1, caractérisé en ce que le rapport molaire 5-nitrofurfural/acétaldéhyde est de 1 : 1 à 1 : 2.

3. Procédé selon la revendication 1 ou 2, caractérisé en ce que l'on opère à des températures entre −10 et +90 °C, plus spécialement entre 10 et 80 °C.